# EUROPEAN PATENT APPLICATION

(11) **EP 4 403 840 A1**
(43) Date of publication of application: **24.07.2024**
(21) Application number: 24152522.9
(22) Date of filing: 18.01.2024
(51) Int. Cl.: F24F 8/117, A61L 9/14, B01D 47/02, F24F 8/125, F24F 8/133, F24F 8/80, F24F 13/28, B01D 47/00, F24F 13/20

(54) **A LIQUID-WASHING AIR PURIFYING AND DISINFECTING DEVICE**

(30) Priority: 19.01.2023 CN 202320118748 U
(71) Applicant: Wang, Zhiye, Xuzhou Jiangsu 221333 (CN); Wang, Yebin, Xuzhou Jiangsu 221333 (CN); Xuzhou Xinkun Electric Co., Ltd, Xuzhou Jiangsu 221300 (CN)
(72) Inventor: Wang, Zhiye, Xuzhou Jiangsu 221333 (CN); Wang, Yebin, Xuzhou Jiangsu 221333 (CN)
(74) Representative: Cabinet Chaillot

(57) **Abstract**

A liquid-washing air purifying and disinfecting device comprises a water storage body, the water storage body is filled with water at the bottom, a first air outlet is arranged on the upper part of its sidewall, and an air inlet is arranged on the middle part of its sidewall; a circulating water device comprises a water pump and an outlet pipe, the water pump is arranged at the center of the bottom of the water storage body, the outlet pipe is vertically arranged, and its bottom is connected to the water pump, the water outlet of the outlet pipe extends upward into the middle of the water storage body; a filtering device comprises multiple annular filter layers, the annular filter layers are located above the water surface, the air inlet is arranged below the annular filter layers and above the water surface.

## Description

### 1. Technical Field

The invention relates to the technical field of air purification equipment manufacturing, in particular to a liquid-washing air purifying and disinfecting device.

### 2. Background Art

The economic and social activities of high-density populations will inevitably produce a large amount of fine particulate matter. Once its emission exceeds the atmospheric circulation capacity and carrying capacity, the concentration of fine particulate matter will continue to accumulate. If affected by cooling weather and other factors at this time, a large-scale haze is prone to occur, severely affecting the air quality of the city.

To improve the air quality in cities, a large number of air purifiers are widely used. Various technologies and media are adopted in existing air purifiers, making it possible to provide users with clean and safe air. Commonly used air purification technologies include adsorption technology, negative (positive) ion technology, catalytic technology, photocatalyst technology, superstructured photomineralization technology, HEPA high-efficiency filtration technology, electrostatic dust collection technology, etc.; material technologies mainly include photocatalyst, activated carbon, synthetic fibers, HEAP high-efficiency materials, negative ion generators, etc. Existing air purifiers are mostly composite types, which means that multiple purification technologies and materials are used simultaneously, resulting in a complex overall structure.

### 3. Summary of the Invention

In view of the above-mentioned problems of the prior art, the invention proposes a liquid-washing air purifying and disinfecting device, which has a compact overall structure, is easy to use and has good filtration effect.

Specifically, the invention proposes a liquid-washing air purifying and disinfecting device, comprising:
A water storage body, the water storage body is filled with water at the bottom, a first air outlet is arranged on the upper part of its sidewall, and an air inlet is arranged on the middle part of its sidewall;
A circulating water device comprises a water pump and an outlet pipe, the water pump is arranged at the center of the bottom of the water storage body, the outlet pipe is vertically arranged, and its bottom is connected to the water pump, the water outlet of the outlet pipe extends upward into the middle of the water storage body;
A filtering device comprises multiple annular filter layers, the annular filter layers are fixed on the outlet pipe, and the annular filter layers are arranged in a horizontal direction, a plurality of the annular filter layers are arranged at intervals in a vertical direction, the annular filter layers are located above the water surface, the air inlet is arranged below the annular filter layers and above the water surface;
An air supply device is arranged at the top of the water storage body and above the filtering device, the air supply device is used to extract air from its bottom and deliver it to the outside of the water storage body through the first air outlet.

According to an embodiment of the invention, the inner side of the annular filter layers is fixed with the outer wall of the outlet pipe through snap-fit, the outer side of the annular filter layers is fixed with the inner wall of the water storage body.

According to an embodiment of the invention, the filtering device also comprises a annular filtering mesh, which is arranged above the annular filter layers and below the air supply device.

According to an embodiment of the invention, it also comprises a covering plate, which is arranged below the filtering mesh and directly above the water outlet for covering the water outlet.

According to an embodiment of the invention, the annular filter layers are provided with a plurality of filter holes, the filter holes are randomly arranged, and at least two of the plurality of filter holes have different pore sizes.

According to an embodiment of the invention, the structures of multiple annular filter layers are exactly the same, and the filter holes adjacent up and down are not aligned.

According to an embodiment of the invention, it also comprises an air inlet pipe that is connected to the air inlet, the air inlet pipe extends inward and downward along the inner wall of the water storage body.

According to an embodiment of the invention, a splash-proof device is arranged in the air inlet pipe to prevent water from flowing out through the air inlet.

According to an embodiment of the invention, the air supply device comprises a wind hood and a centrifugal fan, the wind hood is disc-shaped, and the centrifugal fan is located inside the wind hood, blades are arranged around the centrifugal fan;

A second air outlet is arranged on the wind hood, the second air outlet is located near one side of the first air outlet.

According to an embodiment of the invention, a muffling device is arranged on the wind hood to eliminate the noise generated by the centrifugal fan.

A liquid-washing air purifying and disinfecting device provided by the invention enables the air and water to form an up and down flow, allowing for full contact to remove impurities from the air and effectively purify air. The device has a compact overall structure and is easy to use.

It should be understood that both the foregoing general description and the following detailed description are exemplary and explanatory, and are intended to provide further explanation of the invention as claimed.

### 4. Brief Description of Accompany Drawings

The accompanying drawings are included to provide a further understanding of the invention. They are included and constitute a part of this application. The accompanying drawings illustrate embodiments of the invention and together with the specification to explain the principles of the invention.
FIG. 1 is a schematic diagram of the liquid-washing air purifying and disinfecting device of one embodiment of the invention.
FIG. 2 is a schematic diagram of the annular filter layer of the liquid-washing air purifying and disinfecting device of one embodiment of the invention.

The above accompanying drawings include the following attached drawings marks:
100 liquid-washing air purifying and disinfecting device, 101 water storage body, 102 circulating water device, 103 filtering device, 104 air supply device, 105 water, 106 first air outlet, 107 air inlet, 108 water pump, 109 outlet pipe, 110 water outlet, 111 annular filter layer, 112 annular filtering mesh, 113 covering plate, 114 filter hole, 115 air inlet pipe, 116 splash-proof device, 117 wind hood, 118 centrifugal fan, 119 blade, 120 second air outlet, 121 water surface.

### 5. Specific Embodiment of the invention

It should be noted that, as long as there is no conflict, the embodiments and features in the embodiments can be combined with each other.

The technical schemes in the embodiments of the invention will be clearly and completely described in combination with the accompanying drawings in the embodiments of the invention. Obviously, the described embodiments are only some of the embodiments of the invention, but not all of the embodiments. The following description of at least one exemplary embodiment is merely illustrative in nature and is not intended to limit the invention or its application or uses. Based on the embodiments in this invention, all other embodiments obtained by those of ordinary skill in the art without making creative efforts shall fall within the protection scope of this invention.

It should be noted that the terms used herein are only for describing specific embodiments and are not intended to limit the exemplary embodiments of the invention. As used herein, the singular forms are also intended to include the plural forms unless the context clearly indicates otherwise. Furthermore, it should be understood that when the terms "comprise" and/or "include" are used in this specification, they indicate the presence of features, steps, operations, devices, components and/or combinations thereof.

Unless otherwise specified, the relative arrangement, numerical expression, and value of the components and steps described in these embodiments are not limited to the scope of this invention. At the same time, it should be understood that, for ease of description, the dimensions of the various components shown in the accompanying drawings are not drawn according to actual proportional relationships. Techniques, methods and devices known to those of ordinary skill in the relevant art may not be discussed in detail, but where appropriate, such techniques, methods and devices should be considered as a part of the authorized specification. In all embodiments shown and discussed herein, any specific values should be interpreted as merely exemplary and not as limiting. Therefore, other examples of the exemplary embodiments may have different values. It should be noted that similar reference numerals and letters indicate similar items in the following drawings; thus, once an item is defined in one drawing, it does not require further discussion in subsequent drawings.

In the description of this invention, it should be understood that the orientation or positional relationship indicated by the terms "front", "back", "upper", "lower", "left", "right", "transverse", "longitudinal", "vertical", "horizontal", "top", "bottom" and so on are based on the orientation or positional relationship shown in the accompanying drawings, only for the convenience of describing the invention and simplifying the description. Without explanation to the contrary, these directional words do not indicate or imply that the indicated device or element must have a specific orientation, as well as a specific orientation structure and operation, therefore, it should not be construed as a limitation on the protection scope of the invention. The directional words "inside" and "outside" refer to the inside and outside relative to the outline of each component itself.

For the convenience of description, spatially relative terms can be used here, such as "on...", "above...", "on the upper surface of...", "upper", etc., to describe the spatial relationship between one device or feature and another device or feature as shown in the drawings. It should be understood that spatially relative terms are intended to encompass the orientation of the device as described in the drawing, and its different orientations in use or operation. For example, if the device shown in the drawing is inverted, a device described as "above another device or structure" or "on another device or structure" will be positioned as "below another device or structure" or "under another device or structure". Therefore, the exemplary term "above" can include both "above" and "below" orientations. The device may be otherwise oriented (rotated 90 degrees or at other orientations), and the spatially relative descriptors used herein will be interpreted accordingly.

In addition, it should be noted that the terms of "first", "second" are used to define the components and parts, only to facilitate the distinction between corresponding components and parts. Unless otherwise stated, the above words have no special meaning and therefore cannot be understood as limiting the protection scope of this invention. Furthermore, although the terms used in this invention are selected from commonly known terminology, some of the terms mentioned in this specification may have been chosen by the applicant based on his or her own judgment, and their detailed meanings are explained in the relevant part of this context. In addition, the invention should be understood not only through the actual terms used, but also through the implications of each term.

According to common sense, when water comes into sufficient contact with air, dust impurities in the air naturally adhere to water droplets. Moreover, these dust impurities can act as condensation nucleus for water vapor, water vapor adheres to the surface of dust impurities and form large water droplets. Therefore, allowing water to come into sufficient contact with air can naturally purify the air. People usually think that the air after rainfall is fresh, it is actually due to water removing the dust impurities from the air.

Additionally, water can also remove formaldehyde from the air because formaldehyde contains a hydroxyl group, making formaldehyde readily soluble in water. The chemical formula of formaldehyde is HCOH, and the chemical formula of water is H₂O, H reacts with OH in H₂O to generate new water. Formaldehyde is a polar molecule, and both formaldehyde and water are polar molecules. Based on the principle of "like dissolves like", it is known that formaldehyde can dissolve in water. This example is provided to illustrate that the use of water for air purification is common sense among those skilled in this art, but it is not intended to limiting. In the invention, water is used as the medium for air cleaning. It is easy to understand that other disinfectants or disinfectant substances dissolved in water can also be added to water to form a disinfectant solution. If the disinfectant solution is used instead of water as the medium for air cleaning, it is equally feasible.

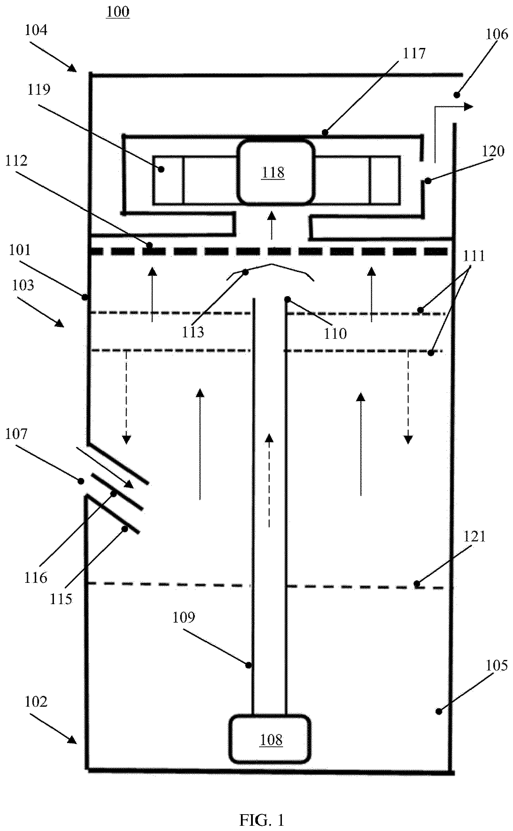

FIG. 1 shows the schematic diagram of the liquid-washing air purifying and disinfecting device of one embodiment of the invention. As shown in the figure, a liquid-washing air purifying and disinfecting device 100 comprises a water storage body 101, a circulating water device 102, a filtering device 103, an air supply device 104.

The water storage body 101 is cylindrical in shape. The water storage body is filled with water 105 for purifying the air at the bottom, a first air outlet 106 is arranged on the upper part of the sidewall of the water storage body 101, and an air inlet 107 is arranged on the middle part of its sidewall.

The circulating water device 102 comprises a water pump 108 and an outlet pipe 109, the water pump 108 is arranged at the center of the bottom of the water storage body 101, the outlet pipe 109 is vertically arranged, and the bottom of the outlet pipe 109 is connected to the water pump 108. The water outlet of the outlet pipe 109 extends upward into the middle of the water storage body 101. The role of the circulating water device 102 is to extract the water 105 from the bottom of the water storage body 101 by using the water pump 108, the water is then transported upward through the outlet pipe 109, and sprayed upward and outward through the water outlet 110, the water 105 naturally flows downward and returns to the bottom of the water storage body 101, forming a continuous circulation.

The filtering device 103 comprises multiple annular filter layers 111, the annular filter layers 111 are fixed on the outlet pipe 109 of the circulating water device 102. The annular filter layers 111 are arranged in a horizontal direction, a plurality of the annular filter layers 111 are arranged at intervals in a vertical direction, the annular filter layers 111 are located above the water surface 121, the air inlet 107 is arranged below the annular filter layers 111 and above the water surface 121. The water 105 sprayed from the water outlet 110 flows downward through the annular filter layers 111 and returns to the bottom of the water storage body 101.

An air supply device 104 is arranged at the top of the water storage body 101 and above the filtering device 103, the air supply device 104 is used to extract air from its bottom and deliver it to the outside of the water storage body 101 through the first air outlet 106.

When the liquid-washing air purifying and disinfecting device 100 is operating, starting the circulating water device 102 and the air supply device 104. The circulating water device 102 extracts the water 105 at the bottom of the water storage body 101 to the top of the annular filter layers 111; under the force of gravity, the water 105 flows back down through the annular filter layers 111 to the bottom of the water storage body 101, forming a continuous circulation, and a water film is formed on the surface of the annular filter layers 111. During this process, external air is introduced above the water surface 121 inside the water storage body 101 through the air inlet 107. The air supply device 104 operates to create a certain negative pressure inside the water storage body 101, causing the air to naturally move upward, the water 105 and the air form an up-and-down convection; the air passes through the annular filter layers 111 and comes into full contact with the water 105, the air is then discharged upwards through the water film bubbling; the purified air is transported outward by the air supply device 104 through the first air outlet 106. The invention provides a liquid-washing air purifying and disinfecting device 100 with a compact overall structure, which allows for sufficient contact between air and water 105 to achieve the object of cleaning and purifying the air.

Preferably, the inner side of the annular filter layers 111 is fixed with the outer wall of the outlet pipe 109 through snap-fit, the outer side of the annular filter layers 111 is fixed with the inner wall of the water storage body 101, this structure facilitates the assembly and fixation of the annular filter layers 111.

Preferably, the filtering device 103 also comprises a annular filtering mesh 112, which is arranged above the annular filter layers 111 and below the air supply device 104. After the air passes upward through the annular filter layers 111, it is then filtered by the annular filtering mesh 112, and then enters the air supply device 104.

Preferably, the liquid-washing air purifying and disinfecting device 100 also comprises a covering plate 113, the covering plate 113 is arranged below the filtering mesh and above the water outlet 110. In the vertical direction, the area of the covering plate 113 is greater than the cross section of the water outlet 110, allowing the covering plate 113 to completely cover the water outlet 110. Starting the circulating water device 102, then the water 105 gushes upward from the water outlet 110 of the outlet pipe 109, and falls downward when it encounters the obstruction of the covering plate 113. The covering plate 113 is shaped with a higher middle and lower edges to allow the water 105 pumped by the water pump 108 to spread outwards. Additionally, the covering plate 113 can guide the upward-moving air to disperse in all directions, facilitating thorough contact between the air and water 105; the air is filtered through the filtering mesh and then is introduced into the air supply device 104.

The annular filter layers 111 are provided with a plurality of filter holes 114, the filter holes 114 are randomly arranged, and at least two filter holes 114 of the plurality of filter holes 114 have different pore sizes.

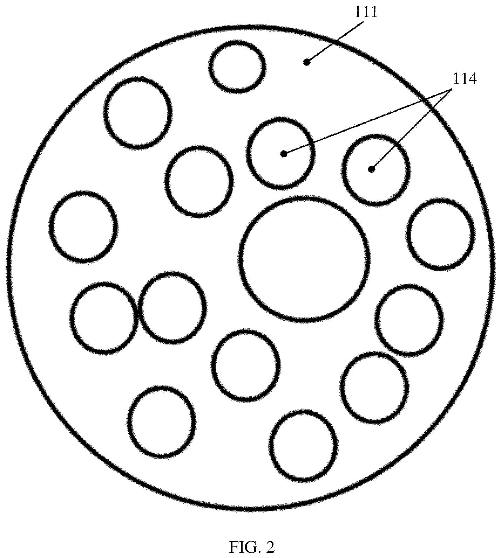

Preferably, multiple annular filter layers 111 are arranged uniformly at equal intervals up and down. It is easy to understand that setting more annular filter layers 111 can bring better purification effects, but it can also affect the air supply efficiency of the air supply device 104. Generally, two or three annular filter layers 111 can be set up for effective air purification. In this embodiment, two annular filter layers 111 are adopted to make air and water 105 more fully in contact. FIG. 2 shows the schematic diagram of the annular filter layer 111 of the liquid-washing air purifying and disinfecting device 100 of one embodiment of the invention. As shown in the figure, the annular filter layers 111 are provided with a plurality of filter holes 114. Preferably, the filter holes 114 on the annular filter layers 111 are irregularly arranged, asymmetrically distributed, and of different sizes. The structures of multiple annular filter layers 111 are the same. The same structure herein refers to the same size of the annular filter layers 111; the size, position and corresponding aperture of the filter holes 114 opened are all the same, which is convenient for industrial production. In terms of installation, each annular filter layer 111 can be rotated by a certain angle along the central axis in the horizontal direction to make the filter holes 114 on the upper and lower annular filter layers 111 staggered from each other in the vertical direction. This design allows the downward flowing water and upward moving air to distribute randomly and evenly, which is equivalent to changing the up and down flow path of the air and water 105 in the vertical direction, and extending the contact time between the air and water 105, thus further enhancing the purification effect of the air and water 105 by contacting.

Preferably, the liquid-washing air purifying and disinfecting device 100 also comprises an air inlet pipe 115, the air inlet pipe 115 is connected to the air inlet 107, the air inlet pipe 115 extends inward and downward along the inner wall of the water storage body 101. This design ensures that the surface of the water storage body 101 has no protrusions, avoiding collision damage and bringing a more compact and practical overall structure.

Preferably, a splash-proof device 116 is arranged in the air inlet pipe 115 to prevent water 105 from flowing out through the air inlet 107.

Preferably, the air supply device 104 comprises a wind hood 117 and a centrifugal fan 118, the wind hood 117 is disc-shaped, and the centrifugal fan 118 is located inside the wind hood 117, blades 119 are arranged around the centrifugal fan 118. Starting the air supply device 104, the blades 119 drive the centrifugal fan 118 to rotate at high speed, and the air is sucked in from the bottom of the centrifugal fan 118. Further, a second air outlet 120 is arranged on the wind hood 117. As is shown in FIG. 1, the second air outlet 120 is located near one side of the first air outlet 106, facilitating the purified air to quickly enter from the second air outlet 120 to the first air outlet 106 and be transported outward.

Preferably, a muffling device is arranged on the wind hood 117 to eliminate the noise generated by the centrifugal fan 118.

The air purification process of the liquid-washing air purifying and disinfecting device 100 will be described in detail below in combination with FIG. 1 and FIG. 2. Referring to FIG. 1, the solid line arrows in the figures indicate the travel path of air. The external air enters above the water surface 121 through the air inlet 107 and the air inlet pipe 115. Under the action of the centrifugal fan 118, the air moves upward, then passes through the filter holes 114 of the two layers of annular filter layers 111, and makes contact with the water film formed on the annular filter layers 111; after bubbling, the air then continues to move upward, and the air in the center encounters the covering plate 113 and spreads around; it is filtered through the filtering mesh, then moves upward and enters into the wind hood 117, enters the second air outlet 120 through the centrifugal fan 118, and then be continuously output to the outside through the first air outlet 106. The dashed line arrows in the figures indicate the circulation route of water 105. The water pump 108 extracts water 105 from the bottom of the water storage body 101 and sprays it upward through the outlet pipe 109; under the obstruction of the covering plate 113, the water is released above the annular filter layer 111 and flows back down through the filter holes 114 of the annular filter layers 111, ultimately returns to the bottom of the water storage body 101. The water 105 and air form an up and down convection, they come into full contact with each other through the annular filter layers 111 to effectively purify the air.

The invention provides a liquid-washing air purifying and disinfecting device 100, which effectively cleans and purifies the air by removing oil fumes, haze, dust, and other pollutants. It has a good purification effect and is particularly suitable for improving urban air quality and enhancing the quality of life. The overall structure is compact.

It will be apparent to those skilled in the art that various modifications and variations can be made to the above-described exemplary embodiments of the invention without departing from the spirit and scope of the invention. Therefore, it is intended that the modifications and variations of the invention fall within the scope of the appended claims and their equivalent technical schemes.

## Claims

1. A liquid-washing air purifying and disinfecting device comprising:
a water storage body, the water storage body is filled with water at the bottom, a first air outlet is arranged on the upper part of its sidewall, and an air inlet is arranged on the middle part of its sidewall;
a circulating water device comprises a water pump and an outlet pipe, the water pump is arranged at the center of the bottom of the water storage body, the outlet pipe is vertically arranged, and its bottom is connected to the water pump, the water outlet of the outlet pipe extends upward into the middle of the water storage body;
a filtering device comprises multiple annular filter layers, the annular filter layers are fixed on the outlet pipe, and the annular filter layers are arranged in a horizontal direction, a plurality of the annular filter layers are arranged at intervals in a vertical direction, the annular filter layers are located above the water surface, the air inlet is arranged below the annular filter layers and above the water surface;
an air supply device is arranged at the top of the water storage body and above the filtering device, the air supply device is used to extract air from its bottom and deliver it to the outside of the water storage body through the first air outlet.

2. The liquid-washing air purifying and disinfecting device of Claim 1, the inner side of the annular filter layers is fixed with the outer wall of the outlet pipe through snap-fit, the outer side of the annular filter layers is fixed with the inner wall of the water storage body.

3. The liquid-washing air purifying and disinfecting device of Claim 1, the filtering device also comprises a annular filtering mesh, which is arranged above the annular filter layers and below the air supply device.

4. The liquid-washing air purifying and disinfecting device of Claim 3, it also comprises a covering plate, which is arranged below the filtering mesh and directly above the water outlet for covering the water outlet.

5. The liquid-washing air purifying and disinfecting device of Claim 1, the annular filter layers are provided with a plurality of filter holes, the filter holes are randomly arranged, and at least two of the plurality of filter holes have different pore sizes.

6. The liquid-washing air purifying and disinfecting device of Claim 5, the structures of multiple annular filter layers are exactly the same, and the filter holes adjacent up and down are not aligned.

7. The liquid-washing air purifying and disinfecting device of Claim 1, it also comprises an air inlet pipe that is connected to the air inlet, the air inlet pipe extends inward and downward along the inner wall of the water storage body.

8. The liquid-washing air purifying and disinfecting device of Claim 7, a splash-proof device is arranged in the air inlet pipe to prevent water from flowing out through the air inlet.

9. The liquid-washing air purifying and disinfecting device of Claim 1, the air supply device comprises a wind hood and a centrifugal fan, the wind hood is disc-shaped, and the centrifugal fan is located inside the wind hood, blades are arranged around the centrifugal fan; a second air outlet is arranged on the wind hood, the second air outlet is located near one side of the first air outlet.

10. The liquid-washing air purifying and disinfecting device of Claim 9, a muffling device is arranged on the wind hood to eliminate the noise generated by the centrifugal fan.
